# EUROPEAN PATENT APPLICATION

(11) **EP 3 165 926 A1**
(43) Date of publication of application: **10.05.2017**
(21) Application number: 15193500.4
(22) Date of filing: 06.11.2015
(51) Int. Cl.: G01N 33/68

(54) **METHOD FOR CHARACTERIZATION OF CELL SPECIFIC MICROVESICLES**

(71) Applicant: Humanitas Mirasole S.p.A., 20089 Rozzano (Milano) (IT)
(72) Inventor: CONDORELLI, Gianluigi, 20089 Rozzano (IT); ANSELMO, Achille, 20089 Rozzano (IT); PAPA, Laura, 20089 Rozzano (IT); VIVIANI ANSELMI, Chiara, 20089 Rozzano (IT)
(74) Representative: Capasso, Olga

(57) **Abstract**

The present invention refers to a method for characterizing and/or measuring and/or sorting the amount of subsets of circulating tissue-derived microvesicles comprising the step of detecting at least one marker selected from the group consisting of: CD172a, CD235a, CD61, CD144, CD14, CD45, CD73, or any combination thereof, on the microvescicles, in an isolated biological sample obtained from the subject using a designed "n x n" antibodies matrix and a sequential stepwise fashion of gating strategy

## Description

### Field of the invention

The present invention relates to a method for characterizing, quantifying and sorting the circulating cell specific microvesicles (MV), in particular the cardiac-derived MV.

The present invention also refers to methods for diagnosing and/or assessing the risk of developing and/or prognosing and/or for monitoring the progression and/or for monitoring the efficacy of a therapeutic treatment and/or for the screening of a therapeutic treatment of a disease, in particular of cardiovascular diseases, by characterizing, quantifying and sorting the circulating tissue-derived microvesicles (MV).

### Background art

Cardiovascular diseases continue to be a leading cause of morbidity and mortality among adults in all economically advanced Countries. The identification of clear biomarkers related to a high risk of cardiovascular diseases remains still largely elusive due to the heterogeneity of the pathological processes underlying these diseases. Therefore, the building of a novel technological approach able to improve the early detection of cardiovascular diseases (CVD) might lead to more accurate interventions and treatment of patients.

Extracellular vesicles (EV) are the key players of the intercellular communications and are released under basal or stress conditions.

EV include apoptotic bodies (ABs), microvesicles (MV) and exosomes, originating from different subcellular compartments [Raposo, et al. J Cell Biol. 2013 Feb 18;200(4):373-83]. In detail, MV are released from their cell of origin upon fusion with the plasma membrane. Noteworthy, the characterization of circulating MV could improve the pathophysiological information for pre-symptomatic disease, leading to a better assessment of patient risk stratification and following therapeutic approaches.

The absence of a method for selectively characterizing and quantifying circulating tissue-specific MV is a limiting factor for applying this technology to disease staging and prediction of acute events.

Therefore, it is still felt the need of a method for selectively characterize and quantify circulating tissue-specific MV, in particular cardiac-derived MV.

### Summary of the invention

The present inventors showed that under stress conditions, circulating cell specific microvesicles (MV), in particular the cardiac-derived MV levels, are a reliable index of the pathological state of the cardiovascular system.

Indeed, the present inventors found a dramatic drop of circulating cardiac- and endothelium-derived MV concentrations after a minimally invasive procedure called Transcatheter Aortic Valve Implantation (TAVI). This "proof of principle" is of pathophysiological interest.

The present inventors developed a simple, accurate and powerful tool for characterizing, quantifying and sorting the circulating tissue-derived MV, focusing on cardiac-derived MVs, for disease prediction, staging and drug-responsiveness.

The inventors based their method on a sequential stepwise fashion of gating strategy using the multicolor Flow Cytometry (FACS). Inventors optimized the FACS analysisto simultaneously characterize, quantify and sorting the circulating tissue-derived MV, including ABs and/or membrane fragments.

The method of the invention may be used for staging of patients with heart failure, cardiomyopathy and valvular diseases and prediction of disease severity in patients with acute coronary artery syndromes, including the drug-responsiveness profile with a prognostic/predictive role on the major adverse cardiac outcome.

Noteworthy, the present method can be set up not only in the strict meaning of CVD, but also in other pathologies as autoimmune diseases (rheumatoid arthritis, arthritis psoriasis, polymyalgia rheumatic, lupus, scleroderma autoimmune disorders, ..).

Cardiovascular complications are the leading negative outcome of autoimmune diseases and could be due to inflammatory status or drug-responsiveness.

Additionally, a wide variety of cancer therapies (i.e. breast cancer, Hodgkin lymphoma, ..) induce the cardiotoxicity, possibly leading to heart failure and other cardiovascular negative consequences.

The method of the invention is highly adaptable to different aspects closely-linked to CVD directly (i.e. heart failure) and/or indirectly (i.e. individual drug-response), with main implications in clinical management and personalized medicine.

The present method allows to accurately discriminate circulating cardiac-derived MV, using the anti CD172a antibody the linking of which to myocardial-derived MV was not previously shown. Conventional biomarkers (as Troponin T (cTnT)) of myocardial injury or heart failure (as NT-proBNP) are mainly used in clinical practice, however they still show a reduced performance in a number of medical pathological conditions.

Commercial magnetic-bead coated with antibody capture, customized dried antibody cocktails for multicolor analysis and/or columns with sized filter cartridges and/or combined with specific antibody filter (SAF) could be applied and developed ad hoc for carrying out the present method.

The present method may be used also in research field for MV sorting. The purified MV subsets can be used for the analysis of their content and function (i.e. proteomics analysis, Next-Generation Sequencing, ..). The obtained findings will improve the knowledge of CVD pathogenesis and drug-responsiveness.

All the antibodies used in the panel designed by the present inventors recognize specific markers expressed on the surface of the MV thus drastically decreasing the background signal typical of the intracellular staining.

The designed matrix 7x7 of the antibodies (Table 1) enables to accurately discriminate the cardiac (CD172a+) circulating tissue-derived MV subsets of interest, strategically placed as last parameter. However, the present matrix is highly adaptable to apply to different study designs and different combinations of antibodies selecting in the last two positions (R13 and R14) circulating tissue-derived MV subsets of major interest and can be designed using different numbers of antibodies ("n x n" matrix), relevant to further clinical applications.

It is in fact possible to substitute one or more antibodies (or add or remove one or more antibodies) of the matrix to characterize and measure the amount of microvesicles released by different organs not only by heart. It is known that organs subjected to stress conditions, e.g. kidney, liver etc, show changes in microvesicle release [Wang Y, et al. Stem Cell Res Ther. 2015 22;6:100][Lemoinne S, at al. Nature Reviews Gastroenterology & Hepatology, 2014 11,350-361][ Ettelaie C,at al Microvasc Res 2008; 76: 152-60][ Royo F, J Extracell Vesicles. 2012 Jul 11;1]

### Detailed description of the invention

It is therefore an object of the invention a method for characterizing and/or measuring the amount of subsets of circulating tissue-derived microvesicles comprising the step of detecting at least one marker selected from the group consisting of: CD172a, CD235a, CD61, CD144, CD14, CD45, CD73, CD3 or any combination thereof, on the microvescicles, in an isolated biological sample obtained from the subject.

Preferably the method of the invention is an ex-vivo or in vitro, more preferably ex-vivo, method.

Preferably the marker to be detected is at least CD 172a.

In a more preferred embodiment of the invention, the markers to be detected are CD172a, CD235a, CD61, CD144, CD14, CD45 and CD73.

The markers may be detected in any order, preferably in the following sequence: CD235a, CD61, CD 144, CD 14, CD45, CD 172a and CD73.

More preferably the markers CD45 and CD 14 are detected at the same time.

In a preferred embodiment of the invention:
- if the microvesicle is positive for CD235a the microvesicle is characterized as an erythroid derived MV;
- if the microvesicle is positive for CD61 the microvesicle is characterized as a platelet derived MV;
- if the microvesicle is positive for CD 144 the microvesicle is characterized as an endothelium-derived MV;
- if the microvesicle is positive for CD14 the microvesicle is characterized as a monocyte-derived MV;
- if the microvesicle is positive for CD45 the microvesicle is characterized as a leukocyte derived MV;
- if the microvesicle is positive for CD172a the microvesicle is characterized a cardiac derived MV;
- if the microvesicle is positive for CD73 the microvesicle is characterized as a stromal/adipocyte derived MV.

In a preferred embodiment, if the microvesicle is positive for CD3 and/or CD45 the microvesicle is a leukocyte-derived MV.

CD3 marker can be used combined with CD45 to better discriminate leukocyte-derived MVs.

A further object of the invention is a method for diagnosing and/or assessing the risk of developing and/or prognosing and/or for monitoring the progression and/or for monitoring the efficacy of a therapeutic treatment and/or for the screening of a therapeutic treatment of a disease in a subject comprising the steps of:
a) characterizing and/or measuring the amount of subsets of circulating tissue-derived microvesicles according to the above defined method;
b) comparing with respect to a proper control and/or reference.

Preferably the above method is an ex-vivo or in vitro method, more preferably an ex-vivo method.

Said disease is preferably selected from the group consisting of: cardiovascular diseases (CVD), autoimmune disease, cancer disease.

In the methods according to the invention, an amount of cardiac- and/or stromal and/or monocyte-and/or endothelium- derived MV, preferably of cardiac-derived MV, in the isolated biological sample obtained from the subject higher or lower than the control amount indicates that the subject is either affected by or is at increased risk for developing cardiovascular diseases (CVD), preferably heart failure, primary or secondary.

In preferred embodiment, an amount of cardiac- and/or stromal and/or monocyte-and/or endothelium- derived MV, preferably of cardiac-derived MV, in the isolated biological sample obtained from the subject higher than the control amount indicates that the subject is either affected by or is at increased risk for developing cardiovascular diseases (CVD), preferably heart failure, primary or secondary.

In a preferred embodiment of the methods of the invention, the measured or characterized subset of circulating tissue-derived microvesicles is the cardiac-derived MV subset.

Said cardiac-derived MV subset is preferably characterized by the presence of the marker CD 172a.

In an alternative method of the invention, cardiac and/or endothelium- and/or stromal-, and/or leukocyte- and/or platelet- and/or monocyte- and/or erythroid -derived MV subset may be characterized by the presence of different markers (or detected with different antibodies) compared to the markers (or antibodies) used in the present method.

Said cardiac-derived microvesicles are preferably negative for at least one of the following markers: CD235a, CD61, CD144, CD14, CD45, CD73, CD3. More preferably said cardiac-derived microvesicles are negative for the following markers: CD235a, CD61, CD144, CD14, CD45, CD73.

In the methods according to the invention, the marker is preferably detected with at least one antibody, or functional fragments thereof, specific for the marker.

Said marker is preferably detected by magnetic beads coated with antibody capture (e.g. MACS® MicroBeads, Miltenyi) and/or customized dried antibody cocktails (e.g. BD Lyotubes) and/or columns with sized filter cartridges and/or combined with specific antibody filter (SAF). In the methods according to the invention, the subsets of circulating tissue-derived microvesicles are preferably characterized and/or their amount measured by means of multicolor flow cytometry technology (FACS), immunogold electron microscopy, immunofluorescence, ELISA, immunoprecipitation, reverse colorimetric immunoassay (RCIA), radioimmune assay (RIA) Electrochemiluminescence, surface plasmon resonance (SPR)-based approach, nanoliter microfluidics (immunoassays) or spectometry.

The cardiovascular disease (CVD) is preferably selected from the group consisting of: aortic stenosis, heart failure, valvular disease, cardiomyopathy, acute coronary artery syndromes, atherosclerosis, myocardial ischemia, infarction, arrhythmias, drug-dependent cardiotoxicity connected to different pathologies (e.g. cancer, HIV-HAART therapies,..).

A further object of the invention is the use of a ligand specific for CD172a for the detection and/or quantification of cardiac-derived MV. Said ligand is preferably an anti-CD172a antibody, or functional fragments thereof

Another object of the invention is a kit comprising detecting means for the markers as above defined, preferably for carrying out the methods as above defined.

Preferably, the CD45 and/or CD14 markers are detected before the detection of the CD172a marker.

The sequence information of the marker CD 172a is recorded in a NCBI-PUBMED database (Gene ID: 140885).

The sequence information of the marker CD235a is recorded in a NCBI-PUBMED database (Gene ID: 2993).

The sequence information of the marker CD61 is recorded in a NCBI-PUBMED database (Gene ID: 3690).

The sequence information of the marker CD144 is recorded in a NCBI-PUBMED database (Gene ID: 1003).

The sequence information of the marker CD14 is recorded in a NCBI-PUBMED database (Gene ID: 929).

The sequence information of the marker CD45 is recorded in a NCBI-PUBMED database (Gene ID: 5788).

The sequence information of the marker CD73 is recorded in a NCBI-PUBMED database (Gene ID: 4907).

The sequence information of the marker CD3 is recorded in a NCBI-PUBMED database (Gene ID: 915/916 and 917).

It is comprised in the methods of the invention the substitution of one or more of the above markers (or the addition or removal of one or more markers). For example, it is possible to modify the present method in order to characterize and measure the amount of microvesicles released by different organs not only by heart, or it is possible to substitute one or more markers with other known markers.

In the same way, it is comprised in the methods of the invention the substitution of one or more of the presently used antibodies (or the addition or removal of one or more antibodies) in order to detect the same described microvesicles or other microvesicles.

The methods of the invention may also be used for identifying the severity of the disease and/or stratifying patients.

The methods of the invention may also be used for sorting the circulating tissue-derived microvesicles (MV).

Microvesicles are circular membrane fragments, ranging in size from 100nm to 1µm in diameter of different subcellular origin released by most known eukaryotic cell types.

In the context of the present invention, the term "extracellular vesicles" comprises apoptotic bodies (ABs), microvesicles (MV) and exosomes.

In the context of the present invention the expression "subsets of circulating tissue-derived microvesicles" refers to different tissue-derived MV. In particular, the subsets are characterized by presenting at least one of the above markers and/or other know markers.

In the context of the present invention, the microvesicle subsets are e.g. erythroid derived MV, platelet derived MV, endothelium-derived MV, monocyte-derived MV, leukocyte derived MV, cardiac derived MV, or stromal/adipocyte derived MV.

A preferred embodiment of the present methods, comprises comparing the amount of each quantified marker to a predetermined cutoff for said marker and determining whether said subject has a risk or is affected, or determining the stage, of cardiovascular disease based on the comparison.

Preferably, a cut-off of cardiac derived MV ≥4.7 (absolute count/mL) was selected to accurately discriminate patients affected by a CVD.

A "severity index" of cardiac-stress may be identified by the skilled man, in particular by applying the present method and cardiac-biomarker on an independent cohort of patients with heart failure and stratified according New York Heart Association (NYHA) class or other classifications.

In the present invention, the control value or proper control may also be selected from a value measured in a healthy patient, a patient affected by a non-CVD, a patient affected by a CVD before a therapeutic treatment, a patient affected by a CVD during the time course of a therapeutic treatment, a patient affected by a CVD at various time point during the course of the disease.

The microvesicles are obtained from any convenient biological sample. Plasma and serum samples from an individual are preferred samples, which may be treated in various ways, including binding to affinity reagents for identification and sorting. For example, samples may be stained with antibodies that selectively bind to the markers.

The sample, e.g. plasma sample, may be subjected to MV isolation with all the methods known to the expert of the art.

The microvesicles may also be sorted and analyzed for the presence of proteins, lipids or nucleic acids of interest, such as RNA, including microRNA.

In the present invention, any combination (e.g. of two, three, four,...) of the above defined markers (or antibodies) may be detected or their amount or alteration measured. The markers may be detected in any sequence.

In the case of a method or a kit for diagnosing and/or assessing the risk of developing and/or prognosing a disease, the proper control may be a sample taken from a healthy patient or from a patient affected by another disorder or pathology, and the control amount may be the amount of the same marker or subset of MV measured in a sample taken from a healthy patient or from a patient affected by another disorder or pathology.

In the case of a method or a kit for monitoring the progression of the disease, the progress of the disease is monitored and the proper control may be a sample taken from the same subject at various times or from another patient, and the control amount may by the amount of the same marker or subset of MV measured in a sample taken from the same subject at various times or from another patient.

If by comparing the measured amount of cardiac- and/or stromal and/or monocyte- and/or endothelium-derived MV, preferably of cardiac-derived MV, with the amount obtained from a control sample, the amount of said subset of MV in the sample isolated from the subject corresponds to an higher or lower, preferably higher, value, the subject may present CVD, may be at risk of developing CVD or go towards an aggravation of said disease.

In the case of a method or a kit for monitoring the efficacy of a therapeutic treatment, the proper control may by a sample taken from the same subject before initiation of the therapy or taken at various times during the course of the therapy and the control amount may be the amount of the same marker or MV subset measured in a sample taken from the same subject before initiation of the therapy or taken at various times during the course of the therapy.

In this case, if the amount of cardiac- and/or stromal and/or monocyte- and/or endothelium-derived MV, preferably of cardiac-derived MV, in the isolated biological sample obtained from the subject is lower or higher, preferably lower, than the control amount, it may indicate that the therapeutic treatment is effective.

In the case of a method or a kit for the screening of a therapeutic treatment, the proper control may be a sample taken from subjects without treatment, or from subjects treated with a substance that is to be assayed or from subjects treated with a reference treatment and the proper control amount may be the average of the amounts of the same marker or cardiac- and/or stromal and/or monocyte- and/or endothelium-derived MV, preferably of cardiac-derived MV, measured in samples taken from subjects without treatment, or from subjects treated with a substance that is to be assayed or from subjects treated with a reference treatment. In this case, if the amount of cardiac- and/or stromal and/or monocyte- and/or endothelium-derived MV, preferably of cardiac-derived MV, in the isolated biological sample obtained from the subject is lower or higher, preferably lower, than the control amount, it may indicate that the tested substance is effective for the treatment of the CVD.

In the context of the present invention, the term "detecting" may be intended also as "measuring the amount". The term "characterizing" may be intended also as "detecting".

Aspects of the invention include analysis of the quality and/or quality (for example the presence of protein or nucleic acid markers of interest) of microvesicles for monitoring cardiac function and therapeutic response in the clinical settings of acute and chronic heart failure.

Aspects of the invention include analysis of the quantity and/or quality (for example the presence of protein or nucleic acid markers of interest) of microvesicles for monitoring of cardiotoxicity responses to drugs, e.g. to cancer therapies (e.g. chemotherapy and radiation therapy). Aspects of the invention include analysis of the quantity and/or quality (for example the presence of protein or nucleic acid markers of interest) of microvesicles for monitoring cardiovascular complications due to autoimmune diseases, inflammatory status, including analysis of the quality and/or quantity for prediction of disease in patients with heart failure and monitoring therapeutic responsiveness.

Aspects of the invention include analysis of the quantity and/or quality (for example the presence of protein or nucleic acid markers of interest) of microvesicles for aortic stenosis following percutaneous aortic valve replacement (TAVI) and other invasive valve replacement procedures and heart failure.

Aspects of the invention include analysis of the quantity and/or quality (for example the presence of protein or nucleic acid markers of interest) of microvesicles for prediction of disease severity in patients with acute coronary artery syndrome or for monitor drug-responsiveness profile.

Such analysis may include detecting the number of microvesicles relative to total serum protein levels, and may include determining the presence of CD 172a on the microvesicles.

Aspects of the invention include analysis of the quantity and/or quality (for example the presence of protein or nucleic acid markers of interest) of microvesicles which is incorporated into a point of care device for purposes of identifying individuals with radiation exposure or specific infections.

In some embodiments, a patient sample, e.g. a plasma or serum sample, is analyzed for the presence of microvesicles, which may be exosomes, comprising markers of interest. Analysis may include mass spectroscopy, but preferably utilizes flow cytometry with the methods of the invention. Markers of interest include CD172a, CD235a, CD61, CD144, CD14, CD45, CD73 or CD3 and any combination thereof.

Assessment in a patient allows improved care, where patients classified according to responsiveness can be treated with an appropriate agent. Patients can be classified upon initial presentation of symptoms, and can be further monitored for status over the course of the disease to maintain appropriate therapy, or can be classified at any appropriate stage of disease progression.

Treatment of particular interest includes pharmacological for heart failure due to primary cardiomyopathy or secondary to various diseases processes, and other invasive replacement procedures including those for cardiac valves, such as TAVI.

In other embodiments of the invention a device or kit is provided for the analysis of patient samples. Alternatively the reagents can be provided as a kit comprising reagents in a suspension or suspendable form, e.g. reagents bound to beads suitable for flow cytometry, preferably magnetic beads coated with antibody capture, or customized dried antibody cocktails for Multicolor analysis and/or columns with sized filter cartridges, and/or combined with specific antibody filter (SAF) and the like. The instructions may comprise instructions for conducting an antibody-based flow cytometry assay.

Detecting means are preferably means able to detect and/or measure the amount of the described markers, e.g. means able to detect the complex antigen-antibody, as enzyme conjugated secondary antibodies, luminescent substrates, magnetic beads coated with antibody capture, customized dried antibody cocktails and/or columns with size filter cartridges and/or combined with specific antibody filter (SAF).

In an embodiment, the method further comprises selecting a therapeutic regimen based on the analysis. In an embodiment, the method further comprises determining a treatment course for the subject based on the analysis.

Marker signature pattern as used herein refers to the spectrum of marker on microvesicles. Once the marker levels and pattern for a particular sample are identified, the data can be used in selecting the most appropriate therapy for an individual. By analysis of marker levels on an individual basis, the specific subclass of disease is determined, and the patient can be classified based on the likelihood to respond to treatments of interest. Thus, the marker signature can provide prognostic information to guide clinical decision making, both in terms of institution of and escalation of therapy as well as in the selection of the therapeutic agent to which the patient is most likely to exhibit a robust response.

The information obtained from the marker profile is used to (a) determine type and level of therapeutic intervention warranted (i.e. more versus less aggressive therapy, monotherapy versus combination therapy, type of combination therapy), and (b) to optimize the selection of therapeutic agents. With this approach, therapeutic regimens can be individualized and tailored according to the specificity data obtained at different times over the course of treatment, thereby providing a regimen that is individually appropriate. In addition, patient samples can be obtained at any point during the treatment process for analysis.

The term "sample" with respect to a patient encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., nucleic acids, polypeptides, etc. The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like.

The term "diagnosis" is used herein to refer to the identification of a molecular or pathological state, disease or condition. The term also includes staging and/or predicting disease severity and/or predicting outcome of the disease.

The term "prognosis" is used herein to refer to the prediction of the likelihood of disease-attributable death or progression, including recurrence and drug resistance of the disease. The term "prediction" is used herein to refer to the act of foretelling or estimating, based on observation, experience, or scientific reasoning. In one example, a physician may predict the likelihood that a patient will survive, following a therapeutic treatment or surgery.

The terms "biomarker," "biomarkers," "marker" or "markers" refer to, without limitation, cytokines, chemokines, growth factors, proteins, peptides, nucleic acids, oligonucleotides, and metabolites, together with their related metabolites, mutations, variants, orthologues, polymorphisms, modifications, fragments, subunits, degradation products, elements, and other analytes or sample-derived measures. Markers can also include mutated proteins, mutated nucleic acids, variations in copy numbers and/or transcript variants. Markers also encompass non-blood borne factors and non-analyte physiological markers of health status, and/or other factors or markers not measured from samples (e.g., biological samples such as bodily fluids), such as clinical parameters and traditional factors for clinical assessments. Markers can also include any indices that are calculated and/or created mathematically. Markers can also include combinations of any one or more of the foregoing measurements, including temporal trends and differences.

To "analyze" includes determining a set of values associated with a sample by measurement of a marker (such as, e.g., presence or absence of a marker or constituent expression levels) in the sample and comparing the measurement against measurement in a sample or set of samples from the same subject or other control subject(s). The markers of the present teachings can be analyzed by any of various conventional methods known in the art. To "analyze" can include performing a statistical analysis to, e.g., determine whether a subject is a responder or a non-responder to a therapy.

A "sample" in the context of the present teachings refers to any biological sample that is isolated from a subject. A sample can include, without limitation an aliquot of body fluid, whole blood, serum, plasma, tissue biopsies, synovial fluid, lymphatic fluid, ascites fluid, and interstitial or extracellular fluid. The term "sample" also encompasses the fluid in spaces between cells, including gingival crevicular fluid, bone marrow, cerebrospinal fluid (CSF), saliva, mucous, sputum, semen, sweat, urine, or any other bodily fluids. "Blood sample" can refer to whole blood or any fraction thereof, including serum and plasma. Samples can be obtained from a subject by means including but not limited to venipuncture, excretion, ejaculation, massage, biopsy, needle aspirate, lavage, scraping, surgical incision, or intervention or other means known in the art.

The term "antibody" may comprise also functional fragments which are able to bind their antigens, as well as molecules comprising such antigen-binding fragments, including engineered antibody fragments, antibody derivatives, bispecific antibodies and other multispecific molecules, scFv, Fv fragment, a Fab fragment, a F(ab)2 fragment, a multimeric antibody, a peptide or a proteolytic fragment containing the epitope binding region.

In a preferred embodiment of the methods of the invention, the antibody used is at least one antibody selected from the group consisting of:
anti-CD235a (GA-R2) (BD Biosciences); [Van Beers EJ, et al. Haematologica.2009 Nov;94(11):1513-9];
anti-CD61 (VI-PL2) (BD Biosciences); [Crompot E, et al. PLoS One. 2015 May 15;10(5):e0127209];
anti-CD144 (REA199) (Miltenyi Biotech); [Koga H, et al. J Am Coll Cardiol. 2005 May 17;45(10):1622-30];
anti-CD45 (HI30) (BD Biosciences) and/or anti-CD 14 (M5E2) (BD Biosciences); [Tähtinen S, et al. Cancer Immunol Res. 2015 May 14] [Griffin JD,et al J. Clin. Invest. 1981; 68: 932-41]; anti-CD73 (AD2) (BD Biosciences); [Müller G, et al.. Obesity (Silver Spring). 2011 Aug;19(8):1531-44];
anti-CD172a (15-414) (eBioscience), [Dubois NC, et al. Nat Biotechnol. 2011 Oct 23;29(11):1011-8],
or any combination thereof.

"Detecting" or "detection", "measuring" or "measurement" in the context of the present teachings refers to determining the presence, absence, quantity, amount, or effective amount of a substance in a clinical or subject-derived sample, including the presence, absence, or concentration levels of such substances, and/or evaluating the values or categorization of a subject's clinical parameters based on a control.

Classification can be made according to predictive modeling methods that set a threshold for determining the probability that a sample belongs to a given class. The probability preferably is at least 50%, or at least 60% or at least 70% or at least 80% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class. The predictive ability of a model can be evaluated according to its ability to provide a quality metric, e.g. AUC or accuracy, of a particular value, or range of values. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC (area under the curve) of at least about 0.7, at least about 0.75, at least about 0.8, at least about 0.85, at least about 0.9, or higher. As is known in the art, the relative sensitivity and specificity of a predictive model can be "tuned" to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about at least about 0.7, at least about 0.75, at least about 0.8, at least about 0.85, at least about 0.9, or higher. Unless otherwise apparent from the context, all elements, steps or features of the invention can be used in any combination with other elements, steps or features.

A sample from an individual is analyzed for the presence of microvesicles, which are optionally detectable labeled for one or more markers of interest. Parameters of interest include microvesicle size, quantity, presence of RNA of interest, presence of proteins of interest, presence of lipids of interest.

When MV are characterized and counted by FACS, a 0.22 to 0.45µm filtered sheath fluid can be used to limit background noise from dust and crystals.

In order to gating MV, beads, e.g. Megamix Plus FSC beads, or propylene non fluorescent different size beads, e.g. NIST Traceable Polystyrene-Melamine Size Standards Nanosphere 0.05µm-1µm, are preferably used.

Preferably, in the methods of the invention, before detecting the above defined markers, ABs and/or membrane fragments are detected.

In the present method, dyes detecting nucleus could be used to detect compromised plasma membranes, e.g. SYTOX, Phallotoxin, Propidium, amino reactive dyes, 7-Aminoactinomycin D etc.

As internal quality check, the inventors investigated the expression of phosphatidylserine on the surface of all the MV-subsets analysed using saturating concentration of Annexin-V. [Heijnen HF, et al Blood. 1999 Dec 1;94(11):3791-9.]

In a preferred embodiment of the invention, only events SYTOX/Phallotoxin double negatives were analyzed for the detection of tissue-derived MVs. Preferably appropriate saturating concentrations of highly specific surface antibodies from 15 to 45 minutes at 4°C or RT were used.

The flow cytometry technology is able to detect the particles size and the granularity by FSC (Forware SCatter) and SSC (Side Scatter) parameters. FSC is a measurement of the amount of the laser beam that passes around the cell. This gives a relative size for the particles.

The SSC parameter is a measurement of the amount of the laser beam that bounces off of particulates inside of the particles.

Using beads of known size one can determine the size of a population based on the FSC parameters.

In a preferred aspect of the invention, membrane fragments while detected, are discarded from the MV analyzed population.

The signature pattern can be generated from a biological sample using any convenient protocol. The readout can be a mean, average, median or the variance or other statistically or mathematically-derived value associated with the measurement. The marker readout information can be further refined by direct comparison with the corresponding reference or control pattern. A binding pattern can be evaluated on a number of points: to determine if there is a statistically significant change at any point in the data matrix; whether the change is an increase or decrease in the binding; whether the change is specific for one or more physiological states, and the like. The absolute values obtained for each marker under identical conditions will display a variability that is inherent in live biological systems and also reflects the variability inherent between individuals.

Following obtainment of the signature pattern from the sample being assayed, the signature pattern is compared with a reference or control profile to make a prognosis regarding the phenotype of the patient from which the sample was obtained/derived. Typically a comparison is made with a sample or set of samples from an unaffected, normal source or the follow up value is considered the own reference value of each patient. Additionally, a reference or control signature pattern can be a signature pattern that is obtained from a sample of a patient known to be responsive or non-responsive to the therapy of interest, and therefore can be a positive reference or control profile.

In certain embodiments, the obtained signature pattern is compared to a single reference/control profile to obtain information regarding the phenotype of the patient being assayed. In yet other embodiments, the obtained signature pattern is compared to two or more different reference/control profiles to obtain more in depth information regarding the phenotype of the patient. For example, the obtained signature pattern can be compared to a positive and negative reference profile to obtain confirmed information regarding whether the patient has the phenotype of interest.

The detection reagents can be provided as part of a kit. Thus, the invention further provides kits for detecting the presence of a panel of specific markers of interest in a biological sample. Procedures using these kits can be performed by clinical laboratories, experimental laboratories, medical practitioners, or private individuals. The kits of the invention for detecting markers comprise affinity reagents useful for generating a prognostic signature pattern, which can be provided in solution or bound to a substrate. The kit can optionally provide additional components that are useful in the procedure, including, but not limited to, buffers, developing reagents, labels, reacting surfaces, means for detection, control samples, standards, instructions, and interpretive information.

Patient outcomes and status can be assessed using imaging-based criteria such as radiographic scores, clinical and laboratory criteria. Multiple different imaging, clinical and laboratory criteria and scoring systems have been and are being developed to assess disease activity and response to therapy in CVD, inflammatory diseases, etc.

A pattern can be obtained as a dataset for an indication of interest. The dataset comprises quantitative data for the presence in serum or other biological sample of at least 1 microvesicle marker, etc. A statistical test will provide a confidence level for a change in the expression, titers or concentration of markers between the test and control profiles to be considered significant, where the control profile can be for selected as appropriate. The raw data can be initially analyzed by measuring the values for each marker, usually in duplicate, triplicate, quadruplicate or in 5-10 replicate features per marker.

A test dataset is considered to be different than a control dataset if one or more of the parameter values of the profile exceeds the limits that correspond to a predefined level of significance.

In the context of the present invention, the term "sensitivity" means the identifying true positives when biomarker levels are used clinically to identify disease/outcome/condition of interest.

In the context of the present invention, the term "specificity" means the identifying true-negatives when biomarker levels are used clinically to identify disease/outcome/condition of interest.

In the context of the present invention, the term "AUC" or "accuracy" means the better tradeoff between false positive and true positive rates to measure predictive accuracy when biomarker levels are used clinically to identify disease/outcome/condition of interest. AUC is determined from ROC curve, a plot of the sensitivity versus (1-specificity) of a biomarker. Additionally, "Accuracy" in the "antibodies matrix" of the present invention means the cross-reactivity and/or co-expression between antibodies inversely linked with Accuracy of the system.

In the context of the present invention, the term "IC95%" ("95% confidence intervals") means an estimated range of values that covers 95% of the normal density curve of a population. On the contrary, the probability of observing a value outside of this area is less than 0.05 (level of significance). Usually all values are estimated at 90% and/or 95% and/or 99% of confidence level, preferable as 95%.

The methods according to the invention are preferably ex-vivo or in vitro methods, more preferably ex-vivo methods.

In the methods of the invention, the subject (or patient) may be an animal or a human, preferably a human.

The invention will be now illustrated by means of non-limiting examples referring to the following figures.

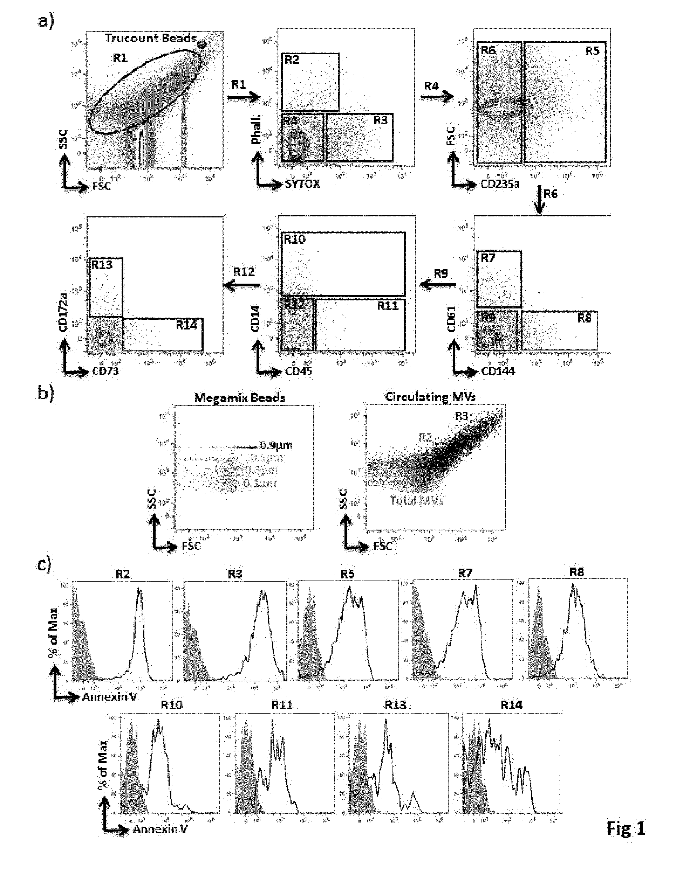

**Figure 1****.** "Stepwise gating strategy" by FACS of circulating tissue-derived MV subsets.
   (a) FACS analysis of plasma derived MV subpopulations assessed by immunostaining. (b) Difference in vesicle size between SYTOX+ apoptotic bodies (R3), cell membrane fragments or apoptotic bodies Phallotoxin+/SYTOX- (R2) and total microvesicles Phallotoxin-/SYTOX-(R5+R7+R8+R10+R11+R13+R14) was confirmed comparing their morphological distribution with Megamix beads at the same FSC/SSC values. Representative images out of three independent experiments.(c) Analysis of Annexin V expression on circulating tissue-derived MV subsets. Images are representative of four analyzed patients.

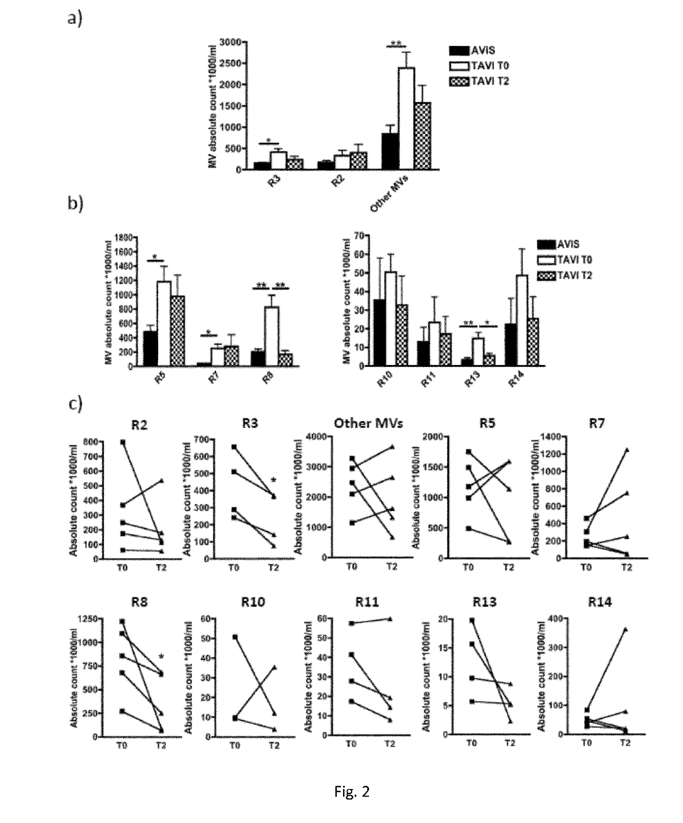

**Figure 2****.** Absolute count of plasma derived microvesicle subpopulations.
   (a-c) Flow cytometric absolute count of SYTOX+ apoptotic bodies (R3), cell membrane fragments or apoptotic bodies Phallotoxin+/SYTOX- (R2) and total microvesicles Phallotoxin-/SYTOX- (R5+R7+R8+R10+R11+R13+R14). (b-c) Flow cytometric absolute count of microvesicle subpopulations R5, R7, R8, R10, R11, R13, and R14. In all panels the MV absolute count was performed using Trucount beads. The analysis were performed on healthy donor (n=4) and TAVI patients before and upon two months of Follow Up (n=3-5). (a-b) Unpaired and (c) paired Wilcoxon test; (*P<0.05; **P<0.01). Error bars represent SEM.

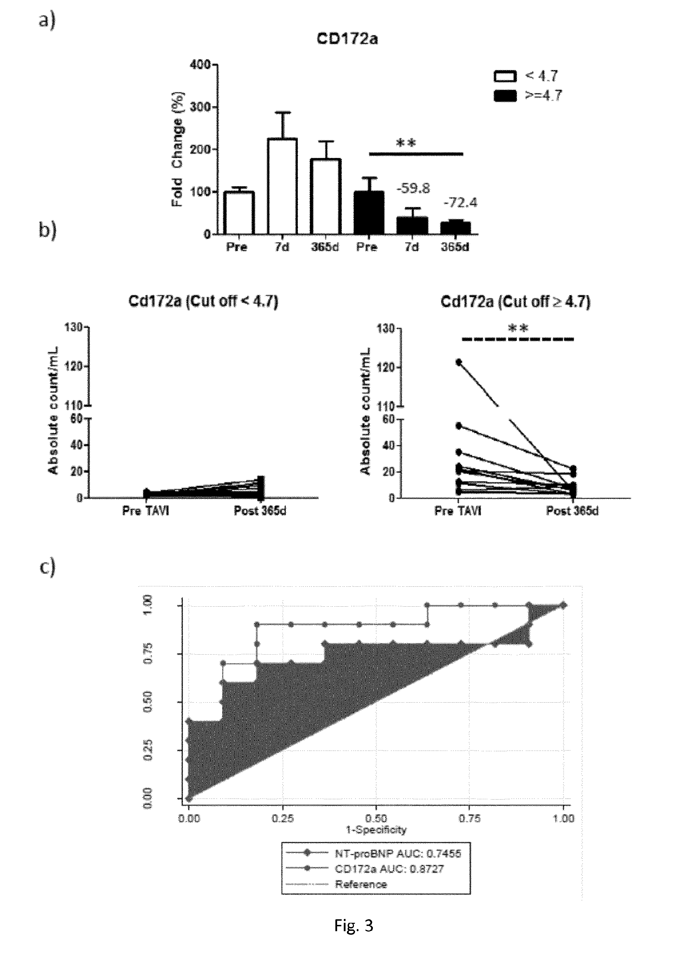

**Figure 3****.** Cardiac-derived microvesicle in AS patients with preselected cut-off.
   (a-b) Flow cytometric absolute count of microvesicle subpopulations R13 in patients with preselected cut-off (CD172a -derived MV absolute count/mL< and ≥4.7). Wilcoxon Matched Pairs analysis was performed on n=10 (≥4.7 ) and n=11 (<4.7). (*P<0.05; **P<0.001). Error bars represent SEM. (c) R13 ROC area against gold standard (NT-proBNP).

### Examples

### Materials and Methods

### Plasma Isolation and storage

According to published recommendations, plasma sampling and storage techniques were standardized for overall patients and healthy subjects.

Informed consent was obtained from each patient and healthy subjects.

A 5 ml sample of peripheral blood was collected in EDTA-containing Vacutainer tubes. The vials were processed within 2 h of collection by centrifugation at 1200 x g at room temperature in a bench top centrifuge for 20 minutes to eliminate all blood cells. To further reduce leukocyte and red cells contamination, the top third of the plasma was aspirated and placed in fresh tubes and frozen at -80°C.

### MV isolation from plasma.

Human plasma was diluted with filtered PBS-/- (no calcium and magnesium) and centrifuged at 500g x 30 minutes at 4°C. The obtained Platelet Free Plasma (PFP) was centrifuged at 12000g x 45 minutes at 4°C. Finally, the supernatant was removed leaving 25µl of a MV-enriched suspension which was further diluted with 75µl of filtered PBS-/-. [Caby MP, et al. Int Immunol. 2005 Jul;17(7):879-87]

### Circulating MV characterization and count by FACS

To limit background noise from dust and crystals, a 0.22 µm filtered sheath fluid was indifferently used preserving sterility, for sample acquisition.

A morphological gate of microvesicles was performed using Megamix Plus FSC beads (0.1 µm to 1 µm beads Biocytex), according to literature [Mobarrez F, et al., Thromb Res. 2010 Mar;125:e110-6].

Events included in a range from 0.1µm to 5µm were clearly discriminated from background noise.

Apoptotic bodies and possible cell membrane fragments derived from freezing/thawing procedure were stained using SYTOX (Invitrogen-Molecular Probes) and Phallotoxin (Invitrogen-Molecular Probes) at room temperature (RT) for 15 and 30 minutes, respectively [Mobarrez F, et al., Thromb Res. 2010 Mar;125:e110-6].

In detail, the SYTOX molecule is a high affinity nucleic acid dye while the Phallotoxin binds F-actin. Both dyes easily penetrates only compromised plasma membranes.

According to the present "stepwise gating strategy", only events SYTOX/Phallotoxin double negatives were analyzed for the detection of tissue-derived MV using the appropriate saturating concentrations of highly specific surface antibodies (30 minutes at RT).

### Tissue-derived MV surface Antibodies selection.

The antibodies were selected according to literature, as following:
- R5 Subset: CD235a (GA-R2) (BD Biosciences); [Van Beers EJ, et al. Haematologica.2009 Nov;94(11):1513-9]
- R7 Subset: CD61 (VI-PL2) (BD Biosciences); [Crompot E, et al. PLoS One. 2015 May 15;10(5):e0127209]
- R8 Subset: CD144 (REA199) (Miltenyi Biotech); [Koga H, et al. J Am Coll Cardiol. 2005 May 17;45(10):1622-30]
- R10+R11 Subset: CD45 (HI30) (BD Biosciences) combined with CD14 (M5E2) (BD Biosciences); [Tähtinen S, et al. Cancer Immunol Res. 2015 May 14] [Griffin JD,et al J. Clin. Invest. 1981; 68: 932-41].
- R14 Subset: CD73 (AD2) (BD Biosciences); [Müller G, et al.. Obesity (Silver Spring). 2011 Aug;19(8):1531-44]
- R13 Subset: CD172a (15-414) (eBioscience), not yet published its involvement in circulating cardiac-derived MVs. [Dubois NC, et al. Nat Biotechnol. 2011 Oct 23;29(11):1011-8]

To avoid potential co-expression of CD172a on monocyte- and leukocyte-MVs derived, as previously reported in the literature, the present method enables to discriminate monocyte- and leukocyte-MVs derived (R10 and R11) in the first steps of the matrix.

All gate regions were restrictively defined using both negative controls: Fluorescence Minus One (FMO) and isotype controls.

Internal MV Quality check: Phosphatidylserine (PS), a marker on the extracellular leaflet of MVs, was verified in all characterized MV-subsets with saturating concentration of Annexin V (BD Biosciences) for 15 minutes at RT.

Circulating MV absolute count was performed using Trucount beads (BD Biosciences), following manufacturer's instructions.

### Results

The present inventors' "stepwise gating strategy", reported in Figure 1A, is a potent and strategic tool able to accurately discriminate the uninvestigated cardiac (R13)-derived MV, minimizing the false positive events and using an anti-CD 172a antibody not known as marker for circulating cardiac-derived MV.

The first assessed gate allows the identification of ABs (R3) and/or membrane fragments (R2). The sequential gating strategy is able to exclude the predominant MV erythroid (R5), platelet (R7), endothelium (R8) and leukocyte (R10 and R11) and to specifically focus on cardiac-(R13) and stromal/adipocyte (R14) -derived MV.

As showed in Figure 1b, these three main subpopulation were size compared with Megamix Plus FSC beads and, as expected, R2 and R3 had a dimension > 0.5µm while the other MVs (R5;R7;R8;R10;R11;R13;R14) ranged between 0.1 and 0.5µm.

Moreover the matrix 7x7 of the antibodies, reported in Table 1, is representative of the applied "stepwise gating strategy". Noteworthy, the high specificity of the antibodies with a cross reaction <10% and accuracy of system (100% for cardiac-derived MV) were successfully reached.

Internal MV Quality check: The Annexin V, a surface marker for the microvesicles [Heijnen HF, et al Blood. 1999 Dec 1;94(11):3791-9.], was evaluated in overall characterized MV subsets, as showed in Figure 1c.

### Preliminary Data on CVD patients

### 1) Pilot Study

Plasma samples were collected from patients with aortic stenosis (AS) at the time of inclusion (time=0) from "Federico II" University, Naples. AS patients were then undergone percutaneous aortic valve replacement (TAVI). Clinical follow-up and plasma sampling was performed at a 2-months follow-up (time=2M). Five TAVI patients and four healthy subjects were recruited. Our "stepwise gating strategy" was applied and the absolute count was summarized in Figure 2. Among the MV characterized from plasma, AB (R3), erythroid-(R5), endothelium- (R8) and cardiac- (R13) derived MV subsets were found at significantly higher levels in pre-TAVI AS patients as compared with healthy donors (Fig.2a and 2b). Strikingly, levels of circulating cardiac- and endothelium-derived MV subsets were significantly reduced after TAVI (Fig.2a and 2b).

Noteworthy, the lower levels of AB (R3) and endothelium- (R8) derived MVs were confirmed applying a Wilcoxon Matched Pairs test (Fig.2c) while the cardiac (R13)-derived MV subset was slightly under the significant threshold, probably due to small sample size.

### 2) Improvement of preliminary data in independent AS patients

The same findings were obtained increasing the sample size with an independent group of AS patients enrolled from the University Hospital of Kiel, Germany. The present "stepwise gating strategy" was applied on 21 pre TAVI AS patients followed at 7 days and 365 days post-surgery. Strikingly, levels of circulating cardiac- and endothelium- derived MV subsets were significantly reduced after 365 days of follow up. Significant Wilcoxon Matched Pairs p-values were obtained. Monocitic- and stromal- derived showed the same significant trends after 1 years post-surgery.

Additionally, the significant interlinking between cardiac, endothelium-, monocitic- and stromal-derived MV was observed by Spearman' rho correlation analysis.

In detail, the interlinked MV-MV showed a rho≥0.80 a P<0.0000, as reported in the correlation matrix of Table II.

The identified inter-linking between MV-MV may characterize also other CVDs.

To optimize and identify a potential "grey zone" of cardiac biomarkers (CD172a) identifying for (R13)-derived MV, a "threshold" analysis was performed. A "quintile-based method" and "Time-based strategy".

A similar cut-off was obtained, as following reported:
- Quintile-based method: cut-off ≥4.7 (absolute count/mL), AUC:0.51 IC95%: 0.34-0.65, Std Error 0.07, Sensitivity (Se): 56% and Specificity (Sp): 48%;

We obtained Se and Sp <0.70 but for biomarkers monitoring disease progression (as well as drug responsiveness) the patient serves as his own control (baseline values versus follow-up values), and the best Se or Sp threshold (>0.7, etc) becomes less important.

R13 Cut-off ≥4.7 (absolute count/mL) was selected to accurately discriminate patients with AS and define the prognosis at 365 days post-TAVI. According to R13 cut-off, we identify the "Grey Zone" of our cardiac-biomarkers (<4.7). The threshold of the cardiac- biomarker into the grey zone has to be optimized with age-matched control group (in progress).

According to previous data about the interlinking of MV-MV, the "best performer cut-off" of cardiac biomarkers (≥4.7) enables to identify the "best performer cut-off" and the "grey zone" of the other correlated MV (endothelium-, monocitic- and stromal-derived MVs).

Wilcoxon Matched Pairs analysis was performed into two obtained groups (CD172a cut-off <

(n=11) and ≥ 4.7 absolute count/mL (n=10), respectively). (Fig.3a-Fig.3b) A strikingly reduction of 72% of CD172a was observed post 365 days.

Finally test equality of ROC area against gold standard (NT-proBNP) was performed in patients with pre-designed CD172a cut-off (≥ 4.7 absolute count/mL). (Fig.3c) Cardiac-biomarkers showed the higher AUC = 0.8727 (Std. Err. 0.08) than gold standard AUC = 0.7455 (Std. Err. 0.12).

**Tables**

| **Table.1 The matrix 7x7 of "Stepwise gating strategy" to characterize circulating tissue-derived MVs from Human Plasma.** | | | | | | | |
|---|---|---|---|---|---|---|---|
| **%** | **CD235a⁺** | **CD61⁺** | **CD144⁺** | **CD14⁺** | **CD45⁺** | **CD172a⁺** | **CD73⁺** |
| **R5** | 100 | 5.5±0.8 | 12.1±4.7 | 2.3±0.8 | 2±0.2 | 2.7±0.7 | 8.6±3.8 |
| **R7** | 0 | 100 | 0 | 1.3±0.3 | 0.3±0.1 | 0.6±0.3 | 1.5±0.4 |
| **R8** | 0 | 0 | 100 | 2.5±0.7 | 2.7±0.6 | 3.3±1.5 | 4±0.8 |
| **R10** | 0 | 0 | 0 | 100 | 3±0.6 | 4.2±1.7 | 1.9±0.8 |
| **R11** | 0 | 0 | 0 | 0 | 100 | 8.7±3.2 | 7±2 |
| **R13** | 0 | 0 | 0 | 0 | 0 | 100 | 0 |
| **R14** | 0 | 0 | 0 | 0 | 0 | 0 | 100 |

Cross reaction or/and co-expression of the antibodies used in the panel were put in a matrix. Data shown are the mean of six samples (± Standard Error).

R5:erythroid-derived; R7: platelet-derived; R8: endothelium-derived; R10: monocyte-derived; R11: leukocyte-derived; R13: cardiac-derived; R14: stromal/adipocyte-derived circulating MVs.

**Table. II**

| | **CD172a** | **CD144** | **CD73** | **CD14** |
|---|---|---|---|---|
| **CD172a** | 1 | | | |
| **CD144** | **Rho=0.81** | 1 | | |
| | P<0.0000 | | | |
| **CD73** | **Rho=0.83** | **Rho=0.92** | 1 | |
| | P<0.0000 | P<0.0000 | | |
| **CD14** | **Rho=0.84** | **Rho=0.94** | **Rho=0.95** | 1 |
| | P<0.0000 | P<0.0000 | P<0.0000 | |

| | | | | |
|---|---|---|---|---|
| Statistical Test: Spearman's rho and P | | | | |

## Claims

1. An ex-vivo or in vitro method for characterizing and/or measuring the amount of subsets of circulating tissue-derived microvesicles comprising the step of detecting at least one marker selected from the group consisting of: CD172a, CD235a, CD61, CD144, CD14, CD45, CD73, CD3 or any combination thereof, on the microvescicles, in an isolated biological sample obtained from the subject.

2. The method according to claim 1 wherein the marker to be detected is at least CD172a.

3. The method according to any one of previous claims wherein the markers to be detected are CD172a, CD235a, CD61, CD144, CD14, CD45 and CD73.

4. The method according to any one of the previous claims wherein
- if the microvescicle is positive for CD235a the microvesicle is characterized as an erythroid derived MV;
- if the microvescicle is positive for CD61 the microvesicle is characterized as a platelet derived MV;
- if the microvescicle is positive for CD 144 the microvesicle is characterized as an endothelium-derived MV;
- if the microvescicle is positive for CD14 the microvesicle is characterized as a monocyte-derived MV;
- if the microvescicle is positive for CD45 the microvesicle is characterized as a leukocyte derived MV;
- if the microvescicle is positive for CD 172a the microvesicle is characterized a cardiac derived MV;
- if the microvescicle is positive for CD73 the microvesicle is characterized as a stromal/adipocyte derived MV.

5. An ex-vivo or in vitro method for diagnosing and/or assessing the risk of developing and/or prognosing and/or for monitoring the progression and/or for monitoring the efficacy of a therapeutic treatment and/or for the screening of a therapeutic treatment of a disease in a subject comprising the steps of:
a) characterizing and/or measuring the amount of subsets of circulating tissue-derived microvesicles according to the method of any of claims 1-4;
b) comparing with respect to a proper control and/or reference.

6. The method according to claim 5, wherein the disease is selected from the group consisting of: cardiovascular diseases (CVD), autoimmune disease, cancer disease.

7. The method according to any one of previous claims, wherein an amount of cardiac- and/or stromal and/or monocyte-and/or endothelium- derived MV, preferably of cardiac-derived MV, in the isolated biological sample obtained from the subject higher or lower than the control amount indicates that the subject is either affected by or is at increased risk for developing cardiovascular diseases (CVD), preferably heart failure, primary or secondary.

8. The method according to any one of previous claims wherein the measured or characterized subset of circulating tissue-derived microvesicles is the cardiac-derived MV subset.

9. The method according to claim 8 wherein the cardiac- derived MV subset is **characterized by** the presence of the marker CD172a.

10. The method according to claim 9 wherein the cardiac derived microvesicles are negative for the following markers: CD235a, CD61, CD144, CD14, CD45, CD73.

11. The method according to any one of the previous claims wherein the marker is detected with at least one antibody, or functional fragment thereof, specific for the marker.

12. The method according to claim 11, wherein the marker is detected by magnetic beads coated with antibody capture and/or customized dried antibody cocktails and/or columns with sized filter cartridges and/or combined with specific antibody filter (SAF).

13. The method according to any one of previous claims wherein the subsets of circulating tissue-derived microvesicles are characterized and/or their amount measured by means of multicolor flow cytometry technology (FACS), immunogold electron microscopy, immunofluorescence, ELISA, immunoprecipitation, reverse colorimetric immunoassay (RCIA), radioimmune assay (RIA) Electrochemiluminescence, surface plasmon resonance (SPR)-based approach, nanoliter microfluidics (immunoassays) or spectometry.

14. The method according to any one of claims 6-13 wherein the cardiovascular disease (CVD) is selected from the group consisting of: heart failure, aortic stenosis, valvular disease, cardiomyopathy, acute coronary artery syndromes, atherosclerosis, myocardial ischemia, infarction, arrhythmias, drug-dependent cardiotoxicity connected to different pathologies (e.g. cancer, HIV-HAART therapies,..).

15. Use of a ligand specific for CD172a for the detection and/or quantification of cardiac-derived MV.

16. Use according to claim 15 wherein the ligand is an anti-CD172a antibody, or functional fragments thereof.

17. A kit comprising detecting means for the marker as defined in any one claims 1-3 for carrying out the method of any one of claims 1-14.
